(19) Europäisches Patentamt

European Patent Office

Office européen des brevets

(11) EP 2 946 192 B1

## (12) EUROPEAN PATENT SPECIFICATION

(45) Date of publication and mention
of the grant of the patent:
**10.02.2021 Bulletin 2021/06**

(21) Application number: **13831954.6**

(22) Date of filing: **10.12.2013**

(51) Int Cl.:
*G01N 21/01* (2006.01)          *G01N 21/76* (2006.01)
*G01N 33/00* (2006.01)          *G01N 21/03* (2006.01)
*G01N 21/05* (2006.01)

(86) International application number:
**PCT/NL2013/050886**

(87) International publication number:
**WO 2014/092569 (19.06.2014 Gazette 2014/25)**

### (54) GAS MEASUREMENT DEVICE AND METHOD

GASMESSUNGSVORRICHTUNG UND VERFAHREN

DISPOSITIF DE MESURE DE GAZ ET PROCÉDÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.12.2012 DE 202012011771 U
22.08.2013 NL 2011329**

(43) Date of publication of application:
**25.11.2015 Bulletin 2015/48**

(73) Proprietor: **KNMI
3732 GK De Bilt (NL)**

(72) Inventor: **ALLAART, Marc
NL-3732 GK De Bilt (NL)**

(74) Representative: **Hoeben, Ferdinand Egon
Allied Patents B.V.
Postbus 1551
1200 BN Hilversum (NL)**

(56) References cited:
**WO-A1-2004/113892**

- **SITNIKOV N M ET AL: "A Chemiluminescent
Balloon-Type Nitrogen Dioxide Meter for
Tropospheric and Stratospheric Investigations
(NaDA)", INSTRUMENTS AND EXPERIMENTAL
TECHNIQUES, KLUWER ACADEMIC
PUBLISHERS-PLENUM PUBLISHERS, NE, vol.
48, no. 3, 1 May 2005 (2005-05-01), pages 400-405,
XP019297130, ISSN: 1608-3180**
- **Y. KONDO ET AL: "Balloon-borne
chemiluminescent sonde for the measurement of
tropospheric and stratospheric nitric oxide",
REVIEW OF SCIENTIFIC INSTRUMENTS, vol. 55,
no. 8, August 1984 (1984-08), page 1328,
XP055117221, ISSN: 0034-6748, DOI:
10.1063/1.1137934**
- **RIDLEY B A ET AL: "An instrument for nitric oxide
measurements in the stratosphere", REVIEW OF
SCIENTIFIC INSTRUMENTS, AIP, MELVILLE, NY,
US, vol. 45, no. 6, June 1974 (1974-06), pages
742-746, XP002476953, ISSN: 0034-6748, DOI:
10.1063/1.1686726**

**Description**

The present invention relates to a gas measuring device which is mobile, such as able to be sent into space, for performing measurements on gases in a gas mixture. The disclosure also relates to a base station for co-action with a mobile gas measuring device, wherein the gas measuring system comprises a base station. The invention further relates to a method for applying a gas measuring device. The disclosure also relates to a data carrier comprising executable instructions for a computer for the purpose of performing steps of a method according to the invention with a base station.

A field of application of the invention relates to an application for carrying the gas measuring device upward to space, at least through the atmosphere to an altitude of several hundred metres or several kilometres, such as 50 km, preferably by means of a weather balloon. It is known per se in other fields, using a photomultiplier tube, to perform measurements relating to the composition of air on the basis of chemiluminescence. In chemiluminescence there is distinct operation in a gas phase or in a liquid phase.

WO 2004/113892 A1 discloses an apparatus and method for the liberation and detection of nitric oxide in human or animal blood. The nitric oxide is liberated from the blood using a chemical reagent or other means within a temperature controlled reaction vessel. Gas phase nitric oxide is transferred from the reaction vessel to a chemiluminescent detector cell in which the nitric oxide is reacted with ozone. The near infrared radiation is then detected by a photomultiplier controlled by suitable control means so as to produce a nitric oxide detection signal allowing the quantitative determination of nitric oxide in blood.

It is known per se that measurements of photons on the basis of a chemiluminescence in a liquid are possible following a processing in a photomultiplier tube. It is an insight of the present inventors that such a tube cannot be applied in the intended application under a probe to be sent into space by means of a weather balloon.

The present invention, the scope of which defined by appended claim 1, provides on the basis of this insight a gas measuring device which is mobile, such as able to be sent into space, for performing a measurement relating to a concentration of a gas, such as nitrogen oxides, such as nitrogen dioxides, in a gas mixture such as air, comprising:

- a reaction chamber suitable for holding a liquid reaction mixture for the purpose of bringing the gas into contact therein with the liquid reaction mixture;
- gas feed means, such as comprising a gas mixture feed tube, for feeding the gas mixture to the reaction chamber;
- gas discharge means, such as comprising a gas mixture discharge tube, for discharging gas from the reaction chamber;
- at least one photoelectric element, such as a photodiode, for converting photons, which are released during a reaction between the gas and the liquid reaction mixture, to electrical energy;
- a processing circuit for providing a determination of a value of the electrical energy generated from the photoelectric element. According to the invention, the gas feed means are arranged to introduce the gas into the reaction chamber at or close to the underside or at least such that an ascent path of the gas in the reaction chamber is possible under the influence of the Archimedean force.

An advantage of a mobile gas measuring device according to the present invention is that, within an objective of the present inventors, it can also be applied for measurements in many air layers by means of a weather balloon. Using such a device it is possible to verify, or help verify, alternative measurements such as for instance those realized by means of a satellite.

A further advantage is that such a mobile gas measuring device can be given a compact form because the measures for measurement purposes can be given a relatively compact and lightweight form compared to the prior art with the photomultiplier tube. Measuring of the photoluminescence is done here by means of a photoelectric element preferably without the intervention of a heavy, voluminous, large and energy-consuming photomultiplier. A further advantage of the mobile gas measuring device is that it can be applied by means of a relatively limited quantity of liquid reaction mixture, this providing for instance a further weight advantage.

In a first preferred embodiment according to the present invention the device comprises moisture control means, such as comprising sealing means, such as an at least twofold cascade of seals, for making parts of the device liquid-tight, and/or the device comprises moisture absorption means. This greatly improves the measuring reliability of the device.

In a further preferred embodiment according to the present invention the reaction chamber extends in elongate manner in vertical direction so as to provide a path for the gas and the reaction liquid to be in contact therein for the purpose of providing a predetermined time period for which a reaction can take place.

According to a further preferred embodiment according to the present invention, the gas measuring device comprises an electronics compartment for providing shielding from light, fluid and/or electromagnetic radiation, wherein the reaction chamber, the photoelectric element and/or the processing circuit are arranged in the electronics compartment.

The advantage hereof is that the electrical determination is not affected by external factors such as ambient light, exogenous liquid and/or electromagnetic radiation from outside, such as a transmitter containable in the device for transmitting data which can be compiled on the basis of the signal for sampling.

Further examples of the shielding from light, also referred to below as light shield, comprise a light-absorbing coating, such as a black coating, preferably matt black coating, or encasing a part of the device with a light-adsorbing encapsulation, such as a black encapsulation, preferably a matt black encapsulation. Further examples of the shielding against fluids are a liquid and gas-impermeable encapsulation, preferably embodied by means of an encapsulation of plastic, iron, steel and/or wood. A further example of shielding from electromagnetic radiation is by means of a Faraday cage, preferably embodied by means of an encapsulation of a conductor, such as metal, such as iron, wherein the encapsulation is embodied in gauze, preferably fine-mesh gauze, more preferably a plate.

The electronics compartment according to the preferred embodiment of the present invention is provided as a compartment having a function for shielding the electronics while also being suitable for having further parts of the invention arranged therein. In the case parts of the electronics are arranged against the reaction chamber, provision is made that for instance parts of the reaction chamber can likewise be arranged in the electronics compartment, preferably by means of a unitary wall or wall part dividing the electronics compartment.

In a further preferred embodiment according to the present invention the electronics compartment is provided with a fluid seal, preferably by means of sealing means which are suitable for providing a seal on the reaction chamber and on a wall of the electronics compartment.

The advantage hereof is that, despite the electronics compartment and the reaction chamber being lined in different materials, they are connected to each other in a manner such that the fluid seal is provided in reliable manner.

The reaction chamber is preferably embodied with the use of plastic or glass. The electronics compartment is preferably embodied by means of sheet iron and/or steel. The fluid seal adheres within the context of the embodiment to both types of material and this preferred embodiment therefore provides the seal for adhesion to both surfaces of the materials of the electronics compartment and reaction chamber.

In a further preferred embodiment according to the present invention the fluid seal of the electronics compartment is provided with a primer and/or sealant, such as a resin, adhesive and/or epoxy, more preferably with tape such as textile tape, preferably impregnated. A cost-effective and/or easily arrangeable embodiment can be realized by means of such materials.

In a further preferred embodiment according to the present invention the device comprises an electronics enclosure for providing an enclosure of at least a recording part of the photoelectric element, which enclosure is preferably arranged inside the electronics compartment, wherein the electronics enclosure, which preferably comprises shrinking sleeve and/or sealant, encloses the electronics for the purpose of shielding the electronics against fluid and/or light influences. By means of such an enclosure the electronics are shielded in advantageous manner from exogenous photons which have penetrated the electronics compartment.

In a further preferred embodiment according to the present invention the device is provided with:

- a reservoir for holding reaction liquid,
- a reaction liquid feed for feeding reaction liquid from the reservoir to the reaction chamber,
- a reaction liquid discharge for discharging the reaction liquid from the reaction chamber, preferably for feeding the reaction liquid back to the reservoir by means of the reaction liquid discharge. An advantage hereof is that replenishment of the reaction liquid is possible by means of the reservoir for the purpose of keeping the reaction liquid functional. Specific provision is hereby made that the pH value is held within a predetermined functional range. The pH is preferably kept alkaline and/or the reaction liquid is prevented from acidifying due to an excess of carbon dioxide. Fresh reaction liquid is fed to the reaction chamber with some regularity or substantially continuously for the purpose of allowing the gas mixture to react with the reaction liquid.

In a further preferred embodiment according to the present invention the device is provided with liquid enclosing means, such as a closure, such as a stopper, and/or sealant, comprising at least one fluid throughfeed channel for enclosing the reaction liquid in the reaction chamber and/or the reservoir. The advantage is that the reaction liquid is enclosed when the stopper is placed and that a filling opening for reaction liquid is provided when the stopper is not placed, while the valve likewise provides throughfeed of fluid.

In a further preferred embodiment according to the present invention the device is provided with liquid enclosing means which comprise a shielding from light and/or electromagnetic radiation and which are preferably connected to the electronics compartment. The advantage hereof is that the opening in which the stopper is placed for the purpose of closing the reaction chamber does not allow passage of light and/or electromagnetic radiation, thereby obviating the problem of measuring results being influenced via a throughfeed opening for gas mixture and/or reaction liquid. An alternative designation is that the wall of the Faraday cage hereby extends over the opening closable by means of the liquid enclosing means.

In a further preferred embodiment according to the present invention the mobile gas measuring device is provided with an earthing assembly for bringing to a common electric potential, such as earthing, at least two parts of the device, such as the gas feed means, the gas discharge means, the reaction liquid feed, the reaction liquid discharge, a gas pump, a reaction liquid pump, reflecting means, the reaction liquid, the reservoir, the reaction chamber and/or the processing circuit. Buildup of static electricity has a disruptive effect on electronics such as the processing circuit and the photoelectric element. In order to prevent the occurrence of static electrical charge as far as possible, at least two, preferably more than two, more preferably all parts of the gas measuring device are brought to the same electric potential. Bringing to the same electric potential takes place by means of electrically connecting the reference potentials of two or more different parts of the gas measuring device to each other. Similarly to earth-coupled devices, this is also referred to as 'earthing' for mobile devices.

The reference potential of the processing circuit is for instance thus levelled with a gas injection member, preferably embodied in metal, for the purpose of providing the same electric potential to the reflecting means, reaction mixture in the reaction chamber and reference potential of the processing circuit.

In a further preferred embodiment according to the present invention the mobile gas measuring device is provided with a single outlet for the gas mixture and the reaction mixture. The surplus reaction liquid and the gas mixture and possible reaction products are hereby discharged in advantageous manner by means of an outlet.

In a further preferred embodiment according to the present invention the mobile gas measuring device is provided with a pressure compensation member. During use a gas mixture is introduced into the gas measuring device. The introduction of the gas mixture increases the pressure in the gas measuring device. The ambient pressure can also vary, such as fall, due to the gas measuring device rising. In order to prevent this pressure increase in the gas measuring device relative to the ambient causing damage to the gas measuring device, this pressure increase is neutralized by means of a pressure compensation member. The pressure compensation member is preferably arranged in the reservoir. The reservoir is more preferably provided with an opening or valve as pressure compensation member for venting purposes. The liquid reservoir also functions as a separator, as gravitational separator for instance under the influence of gravitational force, for liquid and gas. The liquid sinks downward, while the gas accumulates at the top of the reservoir and can be discharged by the venting.

In a further preferred embodiment according to the present invention the mobile gas measuring device is provided with a gas pump. The introduction of the gas mixture into the reaction chamber by means of the gas feed means, such as a hose or tube, from outside the gas measuring device to the reaction chamber is realized in a preferred embodiment with some uniformity with a quantity of gas volume, this having a positive effect on the stability of the measurement. Use is preferably made of a gas pump for the purpose of introducing a uniform quantity of gas volume into the reaction chamber.

In a further preferred embodiment according to the present invention the mobile gas measuring device is provided with a reaction liquid pump. Replenishment of the reaction mixture in the reaction chamber by means of the reaction liquid feed and reaction liquid discharge is performed in a preferred embodiment with a uniform quantity of liquid volume for the purpose of measurement stability. For introducing a uniform quantity of liquid volume into the reaction chamber use is preferably made of a liquid pump, and this liquid pump is more preferably placed in the reaction liquid feed.

In a further preferred embodiment according to the present invention the mobile gas measuring device is provided with at least two photoelectric elements, which are more preferably connected in parallel. A single photoelectric element, such as a photodiode, produces a limited amount of electrical energy. This is a weak electrical input signal, which is easily affected by interference signals, for the processing circuit. A plurality of photodiodes are provided in the gas measuring device for the purpose of stabilizing the electrical input signal of the processing circuit, and the photodiodes are more preferably connected in parallel. By arranging a plurality of photoelectric elements more electrical energy is generated for the signal to the processing circuit. The current generated from the different photodiodes is added together by more preferably connecting the photoelectric elements in parallel.

In a further preferred embodiment according to the present invention the mobile gas measuring device is provided with a reaction chamber, wherein at least one of the wall parts is transparent, wherein more preferably all walls of the reaction chamber are transparent. For capture of the photons the at least one photoelectric element is advantageously arranged at the position of the transparent part of the walls of the reaction chamber in order to capture with the least possible obstruction the photons released from the reaction in the reaction chamber.

In a further preferred embodiment according to the present invention the mobile gas measuring device is provided with reflecting means, such as a reflective surface around the reaction mixture in the reaction chamber. The photoelectric element produces more electrical energy when more photons are captured. In order to increase the indirect capture of photons the reaction chamber is provided with a specular surface for increasing the photons captured by the photoelectric element. In a reaction chamber wherein at least one part of the wall is transparent, at least the transparent wall is preferably combined with placing of a reflective surface, preferably a specular surface, outside the reaction chamber. A specular inert surface, at least inert in the conditions in the reaction chamber, likewise allows placing in the reaction chamber. Of positive influence is that the transparency of the transparent parts is as high as possible in order to prevent loss of photons for the purpose of the measurement.

In a further preferred embodiment according to the present invention the mobile gas measuring device is provided with a photoelectric reference element for measuring the dark current. The photoelectric reference element, such as a photodiode, generates a dark current subject to, among other influences, thermal noise and background radiation whereby an offset in the determination which is caused by the dark current and to which the photoelectric element is also subject, is compensated. The photoelectric reference element supplies the electrical energy to a processing circuit, preferably a second processing circuit, for the purpose of compensating the dark current. Provision is also made that the reference elements are connected anti-parallel to the photoelectric elements. Only one processing circuit is necessary in such an embodiment.

In a further preferred embodiment according to the present invention the mobile gas measuring device is provided with the photoelectric elements and/or the photoelectric reference elements which are arranged in a line or matrix. The photoelectric elements and/or the photoelectric reference elements are preferably arranged in the same manner. An advantage of a line or matrix arrangement is that, when the photons are generated heterogeneously in the reaction chamber, the influence thereof on the measurement is minimized.

In a further preferred embodiment according to the present invention the mobile gas measuring device is provided with a photoelectric element of the first arrangement which is assigned a respective photoelectric reference element of the second arrangement, and these always form an element pair. An advantage hereof is that the influence of a temperature gradient is minimized since the formed element pair has substantially the same temperature. In advantageous manner the influence of the temperature gradient on the dark current is hereby substantially the same for the photoelectric element and the photoelectric reference element of the same element pair.

In a further preferred embodiment according to the present invention the mobile gas measuring device is provided with photoelectric elements and photoelectric reference elements which are used during operation to perform a differential measurement. Already compensating for the dark current during operation further prevents in advantageous manner two signals for sampling from two processing circuits becoming available and having to be further processed. Only one signal for sampling is in this way made available for further processing, whereby only one sampling circuit is necessary. In a further preferred embodiment according to the present invention the mobile gas measuring device is provided with a temperature sensor, preferably for compensating measurement data at any moment on the basis of a characterization of the photoelectric elements at predetermined temperatures, more preferably for compensating, on the basis of the temperature of the reaction liquid and/or gas mixture, the temperature-dependent behaviour of the photoelectric elements, the photoelectric reference elements, the processing circuit, the gas pump and/or the reaction liquid pump. Correction can for instance be made here for an offset of the photoelectric elements at such temperatures. Provision is made for compensation of temperature influences on measurements by the gas measuring device on the basis of measurements of the temperature of the gas measuring device by means of the temperature sensor. The temperature sensor is preferably placed close to or against the reaction chamber for substantially measuring the temperature of the reaction mixture and/or gas mixture. The determination of a gas in a gas mixture using the gas measuring device requires the gas measuring device to perform determinations which can be compensated over a wide range of temperature conditions. Measurements from geographically differing locations close to the Earth's surface with a temperature range of +60°C to -50°C can be compensated by means of such temperature measurements, just as when the gas measuring device is applied in higher air layers with a lower limit for the temperature range of -100°C.

In a further preferred embodiment according to the present invention the reaction chamber and the reservoir are arranged together in a holder, wherein a separating wall preferably defines a division between a relatively small reaction chamber and a relatively large reservoir, such as in a ratio 1:2, preferably 1:3, more preferably 1:4, 1:10, 1:15, 1:20 or larger. Arranging the reaction chamber and the reservoir in a holder reduces the number of connections, and thereby the chance of liquid leakage via such connections.

In a further preferred embodiment according to the present invention the mobile gas measuring device comprises a housing, such as a temperature-insulating box for holding at least the reaction chamber, the photoelectric element and/or the processing circuit for the purpose of substantially excluding weather influences such as variations in temperature and/or air humidity and/or for preventing damage from shocks, such as during a landing on Earth. An advantage of such a housing is that this substantially small temperature drop is enhanced during the measurements.

In the mobile gas measuring device an input of the processing circuit connected to the photoelectric element and/or the photoelectric reference element is more preferably provided with an air insulation for minimizing parasitic resistances. A disruptive influence of a printed circuit board usually in the order of magnitude of the resistors for this application is hereby prevented.

At least a part of the device, at least the reaction chamber and the photoelectric element, is more preferably placed in one or more light seals for substantially light-proof closure of the device. In view of the weak light output from the reaction in the reaction chamber, this measure prevents or minimizes measurement errors due to photons penetrating in undesirable manner.

The gas injection member for introducing the gas mixture into the reaction chamber is preferably fixed by means of a support member, which is, according to the invention, arranged relative to the reaction chamber, so that the gas can be introduced into the reaction chamber at or close to the underside or at least such that an ascent path of the gas in the reaction chamber is possible under the influence of the Archimedean force.

In a further preferred embodiment according to the present invention the mobile gas measuring device comprises a sampling circuit, such as an AD converter, preferably provided with an analog multiplexer and/or a sample&hold circuit, for the purpose of sampling the signal for sampling to preferably a digital signal, which is preferably comprised in a transmitter unit. An advantage is that storing and/or sending of a digital signal provides advantages over storing and/or sending an analog signal, such as preventing loss of signal-noise ratio (SNR).

In a further preferred embodiment according to the present invention the mobile gas measuring device comprises a transmitter unit, preferably a radio transmitter unit, for transmitting data relating to the determinations, and more preferably also comprises a receiver for receiving data such as receipt confirmation data. Equipping the mobile gas measurement device with a radio transmitter unit enables external processing and/or safeguarding of the measurement data from the gas measuring device.

In a further preferred embodiment according to the present invention the mobile gas measuring device comprises a transmitter unit which is functional for transmitting data relating to the ambient temperature and/or pressure. By also transmitting ambient temperature and/or pressure it becomes possible to compensate the sampled values externally for ambient temperature and/or pressure influences.

A further example according to the present disclosure relates to a base station, comprising:

- a receiver for receiving data, such as determinations and other information, from the gas measuring device;
- a processing unit and at least one memory for processing the received determinations. An advantage of applying a base station is that, before landing or loss of the mobile gas measuring device, the determinations are transferable from the mobile gas measuring device to the base station, which comprises for instance a safe storage or a connection to a safe storage.

A further example according to the present disclosure relates to a gas measuring system comprising a mobile gas measuring device according to the present invention and/or a base station according to the present disclosure for receiving data relating to the determined values to be transmitted, such as radiographically, by the mobile gas measuring device. Advantages of this example according to the present disclosure are as described with reference to the above stated embodiments. A further advantage of this example according to the disclosure is that the base station and mobile gas measuring device can exchange data during use, preferably in one direction from the measuring device to the base station, but also in two directions for the purpose of providing reception check and optional retransmission. An alternative is data storage in the measuring device for the purpose of loading data from the gas measuring device following landing.

A further aspect according to the present invention relates to a method for measuring a gas, such as nitrogen oxides, such as nitrogen dioxides, in a gas mixture such as air, applying measurement data obtained by means of a mobile gas measuring device according to the present invention, wherein the method comprises the steps of:

- feeding a gas mixture into a reaction chamber;
- bringing the gas mixture into contact with a reaction liquid in the reaction chamber;
- allowing the gas mixture to react with the reaction liquid for the purpose of generating photons;
- allowing the gas mixture to exit the reaction chamber by means of the gas discharge means;
- capturing the generated photons with a photoelectric element for the purpose of converting photons to electrical energy;
- converting the electrical energy with the processing circuit, such as an amplification circuit for a sampling circuit, for the purpose of providing at least one signal for sampling corresponding to a value of the released photons to a value substantially corresponding to the quantity of gas in the gas mixture.

Advantages of this aspect according to the present invention have already been described with reference to the above stated embodiments.

In a further preferred embodiment according to the present invention the following steps of the foregoing method are augmented with steps of:

- supplementing or replenishing the reaction liquid in the reaction chamber by means of additional reaction liquid via a reaction liquid feed and reaction liquid discharge. The advantage hereof is that acidification of the reaction liquid in the reaction chamber is reduced, as described with reference to the above stated aspects.

In a further preferred embodiment according to the present invention the following steps of the foregoing method are augmented with steps of:

- applying a reaction liquid for use in a reaction with minimal foam production, such as a luminescent liquid, such as liquid containing luminol. The advantage is that a foam-free reaction liquid allows less fluid reaction substance to escape by means of the venting.

In a further preferred embodiment according to the present invention the following steps of the foregoing method are augmented with steps of:

- determining the altitude of the gas measuring device by means of the ambient temperature and/or pressure.

An advantage of determining the altitude of the gas measuring device is that the concentration of a gas at a determined altitude is hereby provided.

A further aspect according to the present invention relates to a method for processing a measurement relating to a concentration of a gas, such as nitrogen oxides, such as nitrogen dioxides, in a gas mixture such as air, applying measurement data obtained by means of a mobile gas measuring device as described above, wherein the method comprises steps for:

- receiving the at least one sampled signal obtained by means of a sampling of the signal for sampling;
- determining on the basis of the sampled signal the electrical energy coming from the at least one photoelectric element, which signal for sampling is suitable for performing further steps appropriate for determining a concentration of a gas in a gas mixture on the basis of the electrical energy.

Advantages of this aspect according to the present invention are as described with reference to the above stated aspects.

In a further preferred embodiment according to the present invention the following steps of the foregoing method are augmented with steps for compensating the concentration of the gas in the gas mixture, wherein one or more of the following compensatable phenomena are compensated:

- dark current, wherein the compensation depends on, among other factors, the temperature;
- chemical effectiveness of a reaction mixture in the gas measuring device, wherein the compensation depends on, among other factors, the temperature and the air pressure;
- compensation of the conversion from particle density to partial pressure, wherein the compensation depends on, among other factors, the temperature and the air pressure;
- compensation of the conversion from partial pressure to the concentration, wherein the compensation depends on, among other factors, the temperature and the air pressure.

The advantage of compensating the measured concentration of a gas is that the concentration of this gas is provided more accurately.

A further example according to the present disclosure relates to a data carrier comprising computer-executable instructions for performing of steps according to the present invention by a base station.

Further advantages, features and details of the present invention will be described in greater detail hereinbelow on the basis of one or more preferred embodiments with reference to the accompanying figures. Similar though not necessarily identical components of different preferred embodiments are designated with the same reference numerals.

Fig. 1 shows a schematic representation of a first preferred embodiment of a mobile gas measuring device according to the present invention.

Fig. 2 shows in vertical section a schematic representation of a detail of a further preferred embodiment, showing inter alia a feed to and discharge from a reaction chamber with sealing means.

Fig. 3 shows a schematic top view of a further preferred embodiment of a mobile gas measuring device in a holder.

Fig. 4 shows a side view of a further preferred embodiment of a mobile gas measuring device.

Fig. 5 shows a top view of a further preferred embodiment of a mobile gas measuring device.

Fig. 6 shows a preferred embodiment of the processing circuit.

Fig. 7 shows a preferred embodiment of the electronics.

Fig. 8 shows a schematic representation of steps which are preferably implemented in software.

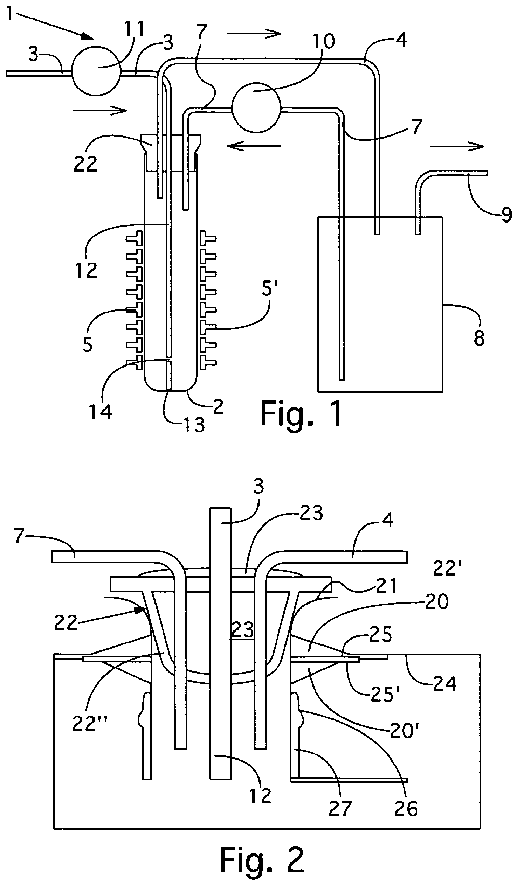

A first preferred embodiment (Fig. 1) according to the present invention relates to a mobile measuring device 1. Device 1 comprises a reaction chamber 2 and a reservoir 8, a discharge 4 and reaction liquid feed 7. Reaction liquid feed 7 connects reservoir 8 in fluid manner to the reaction chamber for feed of liquid from reservoir 8 to reaction chamber 2. A reaction liquid pump 10 is coupled to feed 7 for the purpose of pumping the reaction liquid. Discharge 4 connects the reaction chamber to the reservoir for the purpose of discharging a reaction liquid and a gas mixture.

Reservoir 8 preferably also comprises a vent 9 for venting' the reservoir to a substantially ambient pressure. The reaction chamber according to this embodiment is closable by means of liquid enclosing means which are described in greater detail with reference to figure 2.

Gas feed means 3 are provided with a gas pump 11 for feeding a gas mixture from the outside environment. Gas feed means 3 are connected to a gas injection member 12, such as a gas introducing conduit or pipe, which protrudes from the upper side into reaction chamber 2. Gas feed 3, gas pump 11 and gas injection member 12 are coupled for throughfeed of the gas mixture. The underside of gas injection member 12 is provided with passage openings 14 for passage of gas from the interior of the member to the reaction chamber. The member is also fixed relative to reaction chamber 2 by means of a support member 13, wherein the fixation serves the purpose of providing a continuous and reproducible ascent path of the gas mixture in the reaction chamber during use.

Support member 13 is preferably embodied as a disc, wherein the gas injection member 12 protrudes through the middle and the edge of the disc supports on the reaction chamber wall, or as fixation of an end part of gas injection member 12 to the underside of reaction chamber 2. Support member 13 is preferably arranged in a manner that prevents disruption of the gas mixture in the ascent path in the reaction liquid. Support member 13 is more preferably placed under passage openings 14.

Reaction chamber 2 extends in elongate manner in vertical direction in order to provide an ascent path for the purpose of providing contact along the ascent path between the gas mixture and the reaction liquid, during which contact a reaction can take place.

A photoelectric element 5 and a photoelectric reference element 5' are placed on the outer side of reaction chamber 2. Photoelectric elements 5,5' are preferably embodied as photodiode, avalanche photodiode, phototransistor, a so-called charge coupled device (CCD) recording element and/or photoresistor. At least the part of reaction chamber 2 where the photoelectric elements are placed is transparent or substantially wholly permeable to photons for passage of the photons in the direction of photoelectric element 5.

A plurality of photoelectric elements 5, such as photodiodes, are preferably used to increase the generated electrical energy and to reduce the influences of noise. The photoelectric elements are or can be arranged in one or more matrices or arrays. These photoelectric elements 5 are more preferably connected electrically in parallel in order to provide the greatest possible generated current. The photoelectric reference element 5' serves to provide a compensation measurement on the basis of the dark current. The compensation measurement serves the purpose of compensating for ambient influences, such as a dark current on the basis of properties of the photodiode in the 'light' of the ambient temperature, or cosmic radiation. The number of photoelectric elements 5 is preferably equal to the number of photoelectric reference elements 5'. The photoelectric reference elements 5' are more preferably connected in the same electrical manner as photoelectric elements 5. Provision is still more preferably made that a photodiode and a reference photodiode always form a diode pair.

The mobile gas measuring device 1 is applied within the context of the invention in determining a concentration of a gas in a gas mixture such as air. A reaction liquid is introduced here, preferably uniformly, into the reaction chamber by means of the liquid pump. The gas mixture is also fed by means of gas pump 11 to the underside of the reaction chamber in order to come into contact from there with the reaction liquid, after which the gas mixture bubbles upward subject to the Archimedean force. As it bubbles upward the gas mixture mixes with the reaction liquid, wherein during the mixing a chemiluminescence reaction takes place which generates photons.

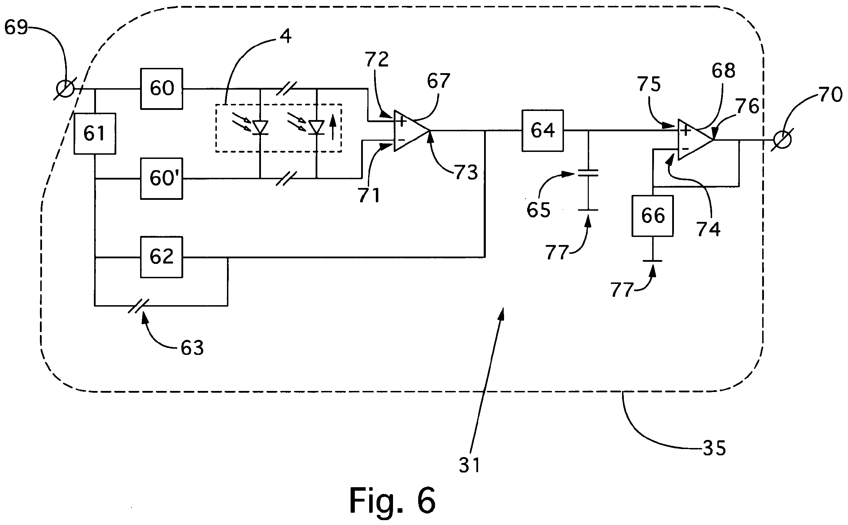

The arrays of photoelectric elements 5 capture the photons in order to convert the photons to electrical energy. A processing circuit 31 (as described further with reference to Fig. 6) amplifies the generated electrical energy from the array 5 and/or the array of photoelectric reference elements 5' and converts these to a signal which can be sampled. The processing circuit preferably takes an electrical form and comprises at least one electrical amplifying element such as a transistor, op-amp or a combination thereof.

In order to prevent acidification of the reaction liquid due to for instance carbon dioxide in the gas mixture, provision is made for replenishment of the reaction liquid. Such replenishment of the reaction liquid can be realized by means of reaction liquid pump 10. Discharge 4 allows discharge of the excess reaction liquid and gas mixture out of reaction chamber 2 to reservoir 8. Pressure compensation member 9, such as a venting tube, provides for the discharge of the excess gas mixture.

An object of using the present invention is to realize a profile comprising a series of measurement data of a determined gas in air at different altitudes. For this purpose the device is preferably launched under a weather balloon in order to carry out a respective number of measurements. When a measurement is recorded for instance every second and the balloon rises at 5 m/s, a profile of measurement data can be built up with 5 metre intervals. These parameters can be freely varied.

The reaction chamber provides space for an ascent path of the gas mixture in the reaction liquid, along which ascent path contact is provided therebetween. The gas mixture is introduced into the bottom of the reaction chamber by means of gas injection member 12. When the reaction mixture is introduced into reaction chamber 2 bubbles are created which rise along the height of reaction chamber 2. Used in this preferred embodiment for introducing the gas mixture into the bottom of reaction chamber 2 is gas injection member 12, also referred to below as bubbler 12, which enters reaction chamber 2 on the upper side and extends to a position close to the underside of reaction chamber 2. The gas mixture comes out of bubbler 12 close to the underside of reaction chamber 2.

In a further preferred embodiment according to the present invention the mobile gas measuring device 1 is provided with a reservoir 8. Addition of $CO_2$ by bringing the reaction liquid into contact with the air reduces the effectiveness of the reaction mixture, such as by causing a decrease in the pH value. When an equal quantity of gas is to be measured in the gas mixture, the photoluminescence reaction will hereby decrease, and the quantity of photons generated will hereby decrease. Increasing and replenishing the reaction mixture increases the time for which the measurement undergoes no or minimal deviation due to for instance acidification of the reaction mixture. The amount of reaction mixture is increased in this embodiment by means of a reservoir 8. The reaction liquid feed 7 and reaction liquid discharge 4 are provided for feed and discharge of the reaction mixture to and from reaction chamber 2.

A second preferred embodiment (Fig. 2) according to the present invention relates to a detail of a mobile gas measuring device 1. The detail figure comprises the following components of the mobile gas measuring device 1: the gas feed means 3, discharge 4 and reaction liquid feed 7. This preferred embodiment displays different levels of sealing/shielding between openings in reaction chamber 2 and the electronics of processing circuit 31.

A first aspect of shielding is the closure, such as a stopper 22, which preferably provides a seal for reaction chamber 2. In alternative manner such a closure is applied on the reservoir or on a combined reaction chamber-reservoir. The stopper comprises a body for passage of conduits, and upper side and a lower side which are provided with openings for passage of feed and discharge means. The stopper preferably comprises a hollow space which is fillable with a sealing agent, such as transparent epoxy 23, for connecting the stopper to the feed and discharge means for the purpose of sealing reaction chamber 2 in order to prevent leakage of the reaction liquid. A sealing agent, such as epoxy, such as transparent epoxy 23, is preferably provided on the upper and/or lower side of the stopper for further connection of the feed and discharge means to stopper 22.

A further level of sealing is performed by means of a sealing mass, such as a fluid which is viscous when applied, such as a paraffin film 21. This paraffin film 21 is situated between stopper 22 and reaction chamber 2.

A second aspect of shielding is provided preferably by means of a separating wall 24 for forming the electronics compartment. This separating wall 24 comprises at least a part of the electronics. Separating wall 24 is provided with a primer or undercoat 25, 25' for providing adhesion between a closing means 20, 20' and separating wall 24. A possible primer is preferably an adhesive tape, such as sticky tape, or a coating such as a paint layer, such as a primer. Closing means 20, 20' closes undesirable fluid openings or leaks between separating wall 24 and the wall of reaction chamber 2. Closing means 20, 20' is preferably epoxy, adhesive, resin and/or sealant.

A third aspect relates to a shielding for the purpose of shielding a recording part of photoelectric element 5 from electromagnetic radiation, light and/or fluid. A sleeve shielding 27 is preferably embodied in sleeve form arranged over reaction chamber 2. Sleeve shielding 27 encloses at least a recording part of photoelectric element 5 between sleeve shielding 27 and the wall of reaction chamber 2. The sleeve shielding is preferably closed on both sides by means of arranging a protective outer end 26. Protective outer end 26 is preferably embodied by means of curing preferably an epoxy, adhesive, resin and/or sealant.

For the purpose of further shielding against light coming from outside, the electronics compartment is provided with a light shield, such as a preferably black coating, and/or a sheet comprising light-absorbing and/or reflecting materials, such as plastics or paper compositions which can be selected within the scope of the invention by the skilled person. Such materials can be arranged on the inner and/or outer side of the electronics compartment.

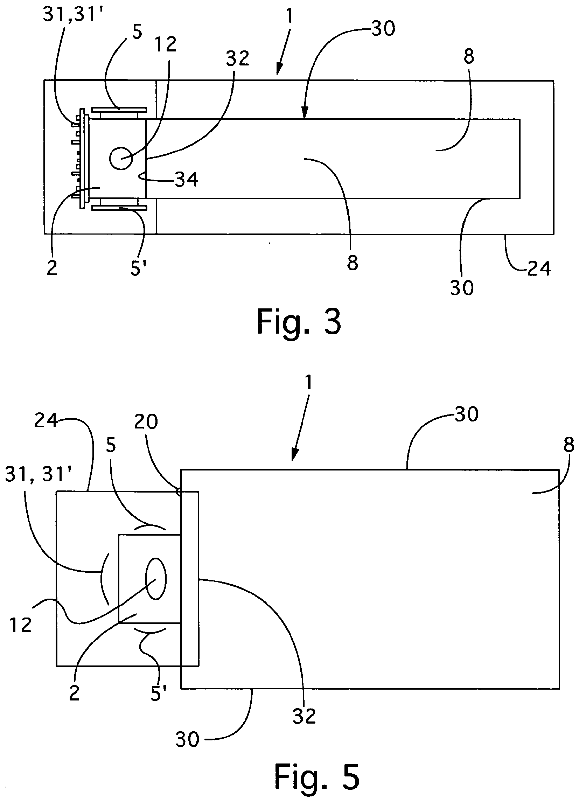

A third preferred embodiment (Fig. 3) according to the present invention comprises a transparent holder 30 in which reservoir 8 and reaction chamber 2 are arranged; this provides a compact arrangement. Holder 30 comprises a reaction chamber 2 and a reservoir 8 arranged adjacently of each other and separated from each other by means of a separating wall 32.

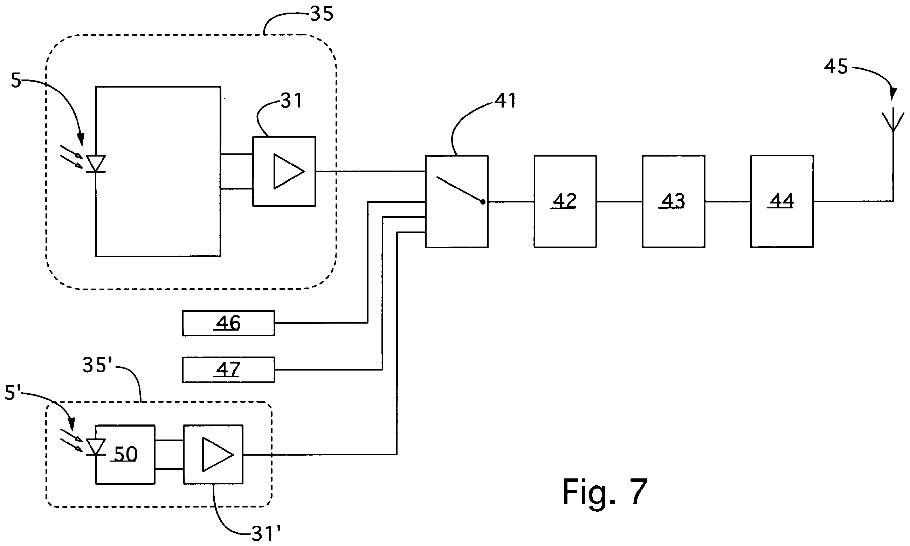

An array of photoelectric elements 5 and an array of photoelectric reference elements 5' are placed on opposite sides of reaction chamber 2 in order to minimize temperature influences. A processing circuit 31,31' is also placed on the outer side of reaction chamber 2. An assembly of the photoelectric elements 5,5' and the circuits 31,31' is described in greater detail or individually below with reference to Fig. 7. Provision is made in this embodiment that signals are processed separately per array of photodiodes by means of a processing circuit. An advantage hereof is that, compared to processing by means of a processing circuit, separately determining a reference voltage is unnecessary. A processing circuit as referred to here is shown in detail in Fig. 6.

Provided for the purpose of increasing the light output are specular members 34 for reflecting photons originating from the reaction for reaching the photodiode by means of reflection. The whole inner surface of the reaction chamber, except for a 'viewing part' of the photodiode, not being the reference photodiode for measuring the dark current, takes a reflective form.

Holder 30 is enclosed by separating wall 24. Separating wall 24 provides the function of a Faraday cage.

The operation of this preferred embodiment is explained with reference to Fig. 5.

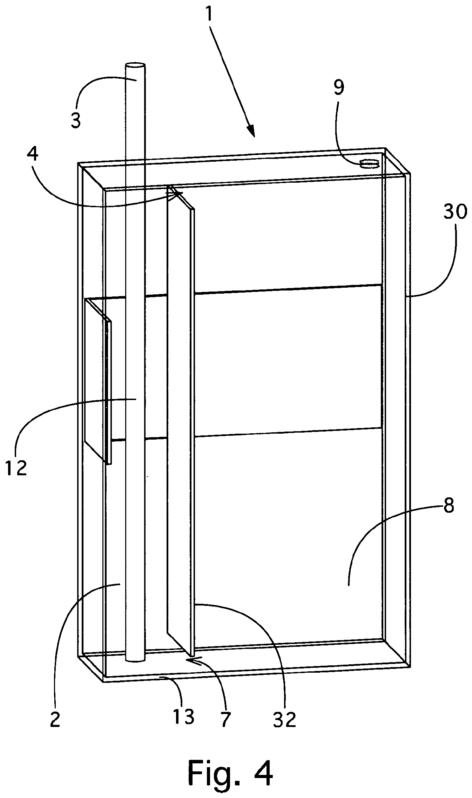

A fourth preferred embodiment (Fig. 4) according to the present invention shows a side view of a mobile gas measuring device 1 as according to fig. 3, wherein a part of the mobile gas measuring device 1 is placed in a holder 30.

Holder 30 takes a transparent form. Holder 30 comprises a reaction chamber 2 and a reservoir 8 which are separated by means of a separating wall 32. Reaction chamber 2 comprises a gas injection member 12. Gas injection member 12 is embodied as a tube with small openings for introducing the gas mixture into reaction chamber 2 at an outer end close to the underside of reaction chamber 2.

Reservoir 8 is connected for gas communication to the outside environment by means of a pressure compensation member 9. Pressure compensation member 9 is embodied as valve on the upper side of reservoir 8 to allow escape of excess substantially gas mixture.

Separating wall 32 extends in vertical direction into the vicinity of the upper side and underside of holder 30. A passage opening 7 on the underside of separating wall 32 and holder 30 is a reaction liquid feed for feeding reaction liquid. A passage opening 4 on the upper side of separating wall 32 and holder 30 is a discharge for discharging reaction liquid and gas mixture. The introduced gas mixture rising in reaction chamber 2 causes a flow of the reaction liquid from bottom to top in reaction chamber 2. Driven by the flow in reaction chamber 2 the reaction liquid circulates by means of discharge 4, reservoir 8 and reaction liquid feed 7. The surplus gas mixture in reservoir 8 is vented by means of valve 9.

A fifth preferred embodiment (Fig. 5) according to the present invention relates to a mobile gas measuring device 1 similar to that according to Fig. 4, wherein a part of the mobile gas measuring device 1 is placed in a holder 30.

Holder 30 comprises a reservoir 8 and a reaction chamber 2. The reservoir and the reaction chamber are separated by separating wall 32. The reaction chamber comprises a gas injection member 12 (shown in Fig. 2). The photoelectric elements 5, photoelectric reference elements 5' and a processing circuit 31 are placed on the outer side of reaction chamber 2, the same as in Fig. 3. The figure also shows a Faraday cage 24 around preferably at least processing circuit 31, photoelectric elements 5 and photoelectric reference elements 5' for the purpose of shielding these components of the mobile gas measuring device from electromagnetic radiation. In this embodiment a side of the Faraday cage 24 is embodied together with separating wall 32. The skilled person will appreciate that in the context of the present invention the Faraday cage likewise extends for shielding purposes along the sides not shown in these views, wherein the cage in Fig. 3 likewise extends substantially as in Fig. 2, and the cage likewise extends in Fig. 2 as is shown in Fig. 3.

A sixth preferred embodiment (Fig. 6) according to the present invention relates to an assembly of processing circuit 31 with the array 5, which together form the sub-circuit 35, in mobile gas measuring device 1. The array 5' with circuit 31', which together form sub-circuit 35', has the same structure for the purpose of connection to the analog multiplexer as shown in Fig. 7.

Processing circuit 31 comprises two op-amps $U_1$ 67, $U_2$ 68. The generated current $I_d$ is generated by means of photodiode arrays $d_1$ to $d_n$ 5 connected in parallel. These photodiodes 5 are placed over inputs 71, 72 of op-amp $U_1$ 67, wherein the anode of photodiodes 5 is connected electrically to the positive input 72 of op-amp $U_1$ 67 and wherein the cathode of photodiodes 5 is connected electrically to the negative input 71 of op-amp $U_1$ 67. Each input 71, 72 of op-amp $U_1$ 67 is also connected electrically to the resistor $R_1$ 60, 60' of 50G'$\Omega$.

The two other sides of resistor $R_1$ 60, 60' are each connected electrically to a side of resistor $R_2$ 61 of 100k'$\Omega$. A side of resistor $R_2$ 61, which is connected by means of resistor $R_1$ 60, 60' to the positive input 72 of op-amp $U_1$ 67, is also connected electrically to the reference voltage $V_{ref}$ 69. The other side of resistor $R_2$ 61 is connected by means of resistor $R_3$ 62 of 1M'$\Omega$ to output 73 of op-amp $U_1$ 67. The reference voltage $V_{ref}$ 69 is equal to 0.5 V. The capacitor $C_1$ 63 of 100nF is arranged over resistor $R_3$ 62.

The output of op-amp $U_1$ 67 is connected electrically to the positive input 75 of op-amp $U_2$ 68 by means of the resistor 64 of 100k'$\Omega$. Positive input 75 of op-amp $U_2$ 68 is also connected electrically to capacitor C2 65 of 10$\mu$F. The other side of this capacitor C2 65 is connected electrically to the zero reference voltage 77. Negative input 74 of op-amp $U_2$ 68 is connected electrically to output 76 of op-amp $U_2$ 68. Negative input 74 of op-amp $U_2$ 68 is also connected electrically to a resistor 66 of 100k'$\Omega$. The electric voltage of output 76 of op-amp $U_2$ 68 is the same as the output voltage 70 of sub-circuit 35.

The current generated from photodiodes 5 can vary between one or several femtoamperes to several milliamperes, preferably between several femtoamperes and several hundred picoamperes. The capacitors $C_1$ 63 and $C_2$ 65 are incorporated for the purpose of filtering the high-frequency interference signals and/or ringing of circuit 31. The reference voltage $V_{ref}$ is applied to prevent the inputs of op-amp $U_1$ 67 starting to float. The reference voltage $V_{ref}$ is selected such that latch-up of op-amp $U_1$ 67 is prevented. Op-amp $U_2$ 68 is connected as a voltage follower and buffers the signal at the input of op-amp $U_2$ 68 in order to provide a voltage $V_{out}$ 70 at the output of op-amp $U_2$ 68 for a further circuit, the preferably provided sampling unit (not shown), which is provided in integrated manner in combination with a digital transmission unit for transmitting the sampled data to an earth receiving station.

The transfer function of sub-circuit 35 is then preferably

$$V_{Out} = I_d\{2R_1(1+R_3/R_2)+R_3\}+V_{Ref} \approx 1,1\cdot10^{12}\cdot I_d + V_{Ref},$$

wherein inter alia a low-frequency variation of $I_d$ is assumed,
wherein amplification factors of op-amps $U_1$ 67 and $U_2$ 68 are of infinite magnitude, and
wherein the resistors $R_1$ 60, 60' at the inputs of op-amp $U_1$ 67 are identical.

A seventh preferred embodiment (Fig. 7) according to the present invention relates to the electronic circuit 40 as can be applied in mobile gas measuring device 1.

Electronic circuit 40 comprises sub-circuit 35 and 35'. Photoelectric element 5 is connected electrically to the inputs of processing circuit 31 and forms together therewith sub-circuit 35. The output of processing circuit 31 is connected electrically to an input of analog multiplexer 41. Photoelectric element 5' is connected electrically to the inputs of processing circuit 31' and forms together therewith sub-circuit 35'. The output of processing circuit 31' is connected electrically to a subsequent input of analog multiplexer 41. Other inputs of analog multiplexer 41 are preferably connected electrically to the other measuring instruments comprised in measuring device 1, such as for air pressure measurements 46 and temperature measurements 47. The output of analog multiplexer 41 is connected electrically to sample&hold circuit 42. The output of sample&hold circuit 42 is connected electrically to the input of an analog-to-digital converter 43 (ADC). The output of the analog-to-digital converter 43 is connected electrically to the input of the transmitting and receiving circuit 44. The output of the transmitting and receiving circuit 44 is connected electrically to antenna 45.

Photoelectric element 5 generates the current $i_d$ which is fed to processing circuit 31 for amplifying the current $i_d$ and converting this current to a voltage. This voltage is fed to analog multiplexer 41. Photoelectric element 5' generates the reference current $i_{d,r}$ which is fed to processing circuit 31' for amplifying reference current $i_{d,r}$ and converting this current to a reference voltage. This reference voltage is fed to analog multiplexer 41. Analog multiplexer 41 selects one of its analog inputs and connects it electrically to its analog output for throughfeed of the input voltage to sample&hold circuit 42. The sample&hold circuit samples a voltage at the input of sample&hold circuit 42. By means of a control input the sample&hold circuit 42 can continue to hold the voltage at its input at an externally determined moment in time at its output for an externally determined period of time. The analog-to-digital converter 43 converts the voltage at the input of analog-to-digital converter 43 to a digitized signal at the output of analog-to-digital converter 43. The transmitting and receiving circuit 44 sends information, such as the digitized signal from analog-to-digital converter 43, by means of an antenna 45. Transmitting and receiving circuit 44 preferably also receives a confirmation that the transmitted information has been correctly received by means of antenna 45.

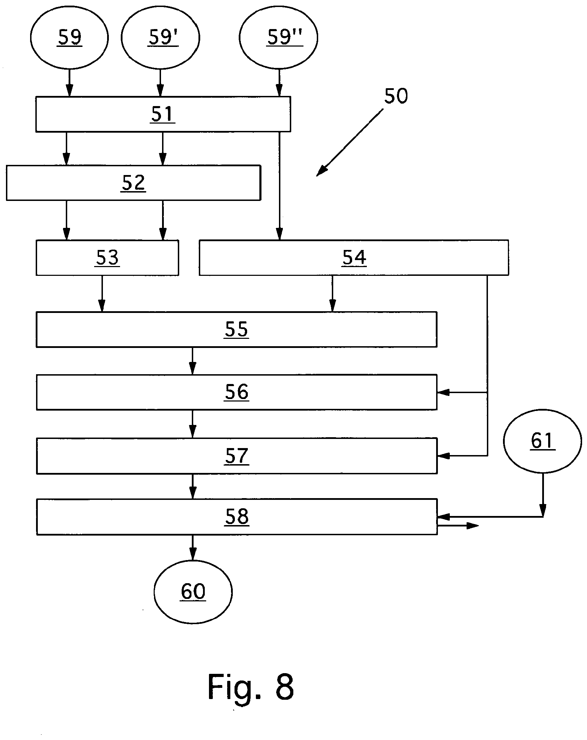

An eighth preferred embodiment (Fig. 8) according to the present invention relates to a schematic overview 50 of the steps preferably implemented in software for performing a determination according to the embodiment of a sampled signal to a concentration of a gas in a gas mixture by means of applying method steps according to the embodiment.

The sampled reference signal 59, the sampled signal 59' and the sampled temperature signal 59" are converted in the first step 51 by means of a step for converting a sampled signal 59, 59', 59" to the voltage associated with the respective sampled signals. The conversion formula is equal to the cascade of the conversion formulae of the analog multiplexer, the sample&hold circuit and/or the analog-to-digital converter.

The thus obtained voltage of the sampled reference signal 59 and the sampled signal 59' are converted in the second step 52 to the respective currents generated by the array of photoelectric elements 5 and by the array of photoelectric reference elements 5'. The conversion formula depends on the selected processing circuit, Fig. 6 showing a preferred embodiment of such a processing circuit 31.

The thus obtained currents are subtracted from each other in the third step 53 in order to obtain the current generated by the captured photons without disruption from influences such as an electronic offset.

In the fourth step 54 the temperature is determined by means of converting the voltage associated with the sampled temperature signal 59". The temperature is preferably applied to carry out compensations on the following steps. The conversion formula of step 54 is the same as a conversion formula predetermined by means of calibration.

The current thus obtained from step three 53 is compensated in the fifth step 55 by means of a compensation formula for the temperature-dependent dark current from photoelectric element 5 and photoelectric reference element 5'.

The current thus obtained from step five 55 is converted in the sixth step 56 by means of the conversion formula for the particle density. The conversion of the current to the particle density by means of a conversion formula is at least dependent on the chemical effectiveness of the reaction mixture and/or the efficiency of photon capture by photoelectric element 5. The chemical effectiveness is at least dependent on the flow rate of the reaction mixture, the flow rate of the gas mixture and/or the temperature of the reaction mixture.

The particle density thus obtained from step six 56 is converted in the seventh step 57 to a partial pressure by means of a conversion formula. The dependency of the conversion formula comprises the dependency on the reaction mixture, the flow rate of the gas mixture, the form of the gas measuring device, such as the form of the reaction chamber 2 and/or pressure compensation member 9, a temperature and/or an air pressure 61.

The partial pressure thus obtained from step seven 57 is converted in the eighth step 58 to a concentration 60 of the gas in the gas mixture by means of a conversion formula. The dependency of the conversion formula comprises the dependency on the temperature and/or air pressure 61.

The present invention has been described in the foregoing on the basis of several preferred embodiments. Different aspects of different embodiments are deemed described in combination with each other, wherein all combinations which can be deemed by a skilled person in the field as falling within the scope of the invention, defined by the appended claims, on the basis of reading of this document are included. These preferred embodiments are not limitative for the scope of protection of this document. The rights sought are defined in the appended claims.

**Claims**

1. Gas measuring device (1) which is mobile, such as able to be sent into space, for performing a measurement relating to a concentration of a gas, such as nitrogen oxides, such as nitrogen dioxides, in a gas mixture such as air, comprising:

   - a reaction chamber (2) suitable for holding a liquid reaction mixture for the purpose of bringing the gas into contact therein with the liquid reaction mixture;
   - gas feed means (3), such as comprising a gas mixture feed tube, for feeding the gas mixture to the reaction chamber (2);
   - gas discharge means (4), such as comprising a gas mixture discharge tube, for discharging gas from the reaction chamber (2);
   - at least one photoelectric element (5), such as a photodiode, for converting photons, which are released during a reaction between the gas and the liquid reaction mixture, to electrical energy;
   - a processing circuit (31), such as an amplification circuit for a sampling circuit, for the purpose of providing a signal for sampling corresponding to a value of the released photons, wherein the gas feed means are arranged to introduce the gas into the reaction chamber (2) at or close to the underside or at least such that an ascent path of the gas in the reaction chamber is possible under the influence of the Archimedean force.

2. Gas measuring device (1) as claimed in claim 1, wherein the device (1) comprises moisture control means, such as comprising sealing means, such as an at least twofold cascade of seals, for making parts of the device (1) liquid-tight, and/or wherein the device (1) comprises moisture absorption means.

3. Gas measuring device (1) as claimed in claim 1, wherein the gas measuring device (1) comprises an electronics compartment (24) for providing shielding from light, fluids and/or electromagnetic radiation, wherein the reaction chamber, the photoelectric element and/or the processing circuit are arranged in the electronics compartment (24), preferably wherein the electronics compartment is provided with a fluid seal, preferably by means of sealing means which are suitable for providing a seal on the reaction chamber and on a wall of the electronics compartment (24).

4. Gas measuring device (1) as claimed in one or more of the foregoing claims, comprising an electronics enclosure for providing an enclosure of at least a recording part of the photoelectric element (5), which enclosure is preferably arranged inside the electronics compartment, wherein the electronics enclosure, which preferably comprises shrinking sleeve and/or sealant, encloses the electronics for the purpose of shielding the electronics against fluid and/or light influences.

5. Gas measuring device (1) as claimed in one or more of the foregoing claims, wherein the device (1) comprises at least two photoelectric elements (5) which are connected in parallel.

6. Gas measuring device (1) as claimed in one or more of the foregoing claims, wherein the reaction chamber (2) is provided with reflecting means, such as a reflective surface, such as a specular surface, for increasing the efficiency of photon recording by the photoelectric elements (5).

7. Gas measuring device (1) as claimed in one or more of the foregoing claims, comprising a photoelectric reference element (5') which is shielded from photons, in particular the photons resulting from the reaction, for measuring a substantially dark current, wherein the number of photoelectric reference elements (5') preferably equals the number of photoelectric elements (5).

8. Gas measuring device (1) as claimed in claim 7, wherein the photoelectric elements (5) and/or the photoelectric reference elements (5') are arranged in a line or matrix, preferably wherein the arrangement is the same for the photoelectric elements (5) and/or photoelectric reference elements (5'), preferably wherein a photoelectric element (5) of the first arrangement is assigned a respective photoelectric reference element (5') of the second arrangement, and these always form an element pair, further preferably wherein the photoelectric elements (5) and photoelectric reference elements (5') are used during operation to perform a differential measurement, further preferably wherein an input of the processing circuit (31) connected to the photoelectric element (5) and/or the photoelectric reference element (5') is provided with an air insulation for minimizing parasitic resistances.

9. Method for performing a measurement relating to the concentration of a gas, such as nitrogen oxides, such as nitrogen dioxides, in a gas mixture such as air, applying measurement data obtained by means of a gas measuring device (1) as claimed in one or more of the foregoing claims 1 to 8, wherein the method comprises the steps of:

- feeding a gas mixture into a reaction chamber (2);
- bringing the gas mixture into contact with a reaction liquid in the reaction chamber (2);
- allowing the gas mixture to react with the reaction liquid for the purpose of generating photons;
- allowing the gas mixture to exit the reaction chamber (2) by means of the gas discharge means;
- capturing the generated photons with a photoelectric element (5) for the purpose of converting photons to electrical energy;
- converting the electrical energy with the processing circuit (31), such as an amplification circuit for a sampling circuit, for the purpose of providing at least one signal for sampling corresponding to a value of the released photons.

10. Method as claimed in claim 9, comprising the steps of:

- supplementing or replenishing the reaction liquid in the reaction chamber (2) by means of additional reaction liquid via a reaction liquid feed (7) and a reaction liquid discharge (4).

11. Method as claimed in claim 9 or 10, comprising the steps of:

- applying a reaction liquid for use in a reaction with minimal foam production, such as a luminescent liquid, such as containing luminol.

12. Method as claimed in one or more of the claims 9 to 11, comprising the steps of:

- determining the altitude of the gas measuring device by means of the ambient temperature and/or pressure.

13. Method for processing a measurement relating to a concentration of a gas, such as nitrogen oxides, such as nitrogen dioxides, in a gas mixture such as air, applying measurement data obtained by means of a gas measuring device (1) as claimed in one or more of the foregoing claims 1 to 8, wherein the method comprises the steps of:

- receiving the at least one sampled signal obtained by means of a sampling of the signal for sampling;
- determining on the basis of the sampled signal the electrical energy coming from the at least one photoelectric element, which signal for sampling is suitable for performing further steps appropriate for determining a concentration of a gas in a gas mixture on the basis of the electrical energy.

14. Method as claimed in claim 13, comprising steps for compensating the concentration of the gas in the gas mixture, wherein one or more of the following compensatable phenomena are compensated:

- dark current, wherein the compensation depends on, among other factors, the temperature;
- chemical effectiveness of a reaction mixture in the gas measuring device, wherein the compensation depends on, among other factors, the temperature and the air pressure;
- compensation of the conversion from particle density to partial pressure, wherein the compensation depends on, among other factors, the temperature and the air pressure;
- compensation of the conversion from partial pressure to the concentration, wherein the compensation depends on, among other factors, the temperature and the air pressure.

**Patentansprüche**

1. Gasmessvorrichtung (1), die mobil ist, wie etwa, um in den Weltraum geschickt zu werden, zum Durchführen einer Messung in Bezug auf eine Konzentration eines Gases, wie etwa Stickoxide, wie etwa Stickstoffdioxide, in einem Gasgemisch wie etwa Luft, Folgendes umfassend:

   - eine Reaktionskammer (2), die zum Halten eines flüssigen Reaktionsgemisches zum Zweck des Inberührungbringens des Gases darin mit dem flüssigen Reaktionsgemisch geeignet ist;
   - Gaszufuhrmittel (3), wie etwa umfassend ein Gasgemisch-Zufuhrrohr, zum Zuführen des Gasgemisches zu der Reaktionskammer (2);
   - Gasausstoßmittel (4), wie etwa umfassend ein Gasgemisch-Ausstoßrohr, zum Ausstoßen von Gas aus der Reaktionskammer (2);
   - wenigstens ein photoelektrisches Element (5), wie etwa eine Photodiode, zum Umwandeln von Photonen, die während einer Reaktion zwischen dem Gas und dem flüssigen Reaktionsgemisch freigesetzt werden, in elektrische Energie;
   - eine Verarbeitungsschaltung (31), wie etwa eine Verstärkungsschaltung für eine Abtastschaltung, zum Zweck des Bereitstellens eines Signals zum Abtasten entsprechend einem Wert der freigesetzten Photonen, wobei die Gaszufuhrmittel angeordnet sind, um das Gas in die Reaktionskammer (2) an oder nahe der Unterseite einzuleiten, oder wenigstens derart, dass ein Aufstiegspfad des Gases in der Reaktionskammer unter dem Einfluss der archimedischen Kraft möglich ist.

2. Gasmessvorrichtung (1) nach Anspruch 1, wobei die Vorrichtung (1) Feuchtigkeitssteuerungsmittel umfasst, wie etwa Dichtungsmittel, wie etwa eine wenigstens zweifache Kaskade von Dichtungen, um Teile der Vorrichtung (1) flüssigkeitsdicht zu machen, und/oder wobei die Vorrichtung (1) Feuchtigkeitsabsorptionsmittel umfasst.

3. Gasmessvorrichtung (1) nach Anspruch 1, wobei die Gasmessvorrichtung (1) ein Elektronikfach (24) zum Bereitstellen von Schutz vor Licht, Fluiden und/oder elektromagnetischer Strahlung umfasst, wobei die Reaktionskammer, das photoelektrische Element und/oder die Verarbeitungsschaltung in dem Elektronikfach (24) angeordnet sind, vorzugsweise wobei das Elektronikfach mit einer Fluiddichtung versehen ist, vorzugsweise mittels Dichtungsmitteln, die zum Bereitstellen einer Dichtung an der Reaktionskammer und an einer Wand des Elektronikfachs (24) geeignet sind.

4. Gasmessvorrichtung (1) nach einem oder mehreren der vorhergehenden Ansprüche, umfassend ein Elektronikgehäuse zum Bereitstellen eines Gehäuses von wenigstens einem Aufzeichnungsteil des photoelektrischen Elements (5), wobei das Gehäuse vorzugsweise in dem Elektronikfach angeordnet ist, wobei das Elektronikgehäuse, das vorzugsweise eine Schrumpfhülse und/oder ein Dichtungsmittel umfasst, die Elektronik zum Zweck des Schutzes der Elektronik gegen Fluid- und/oder Lichteinflüsse einschließt.

5. Gasmessvorrichtung (1) nach einem oder mehreren der vorhergehenden Ansprüche, wobei die Vorrichtung (1) wenigstens zwei photoelektrische Elemente (5) umfasst, die parallel geschaltet sind.

6. Gasmessvorrichtung (1) nach einem oder mehreren der vorhergehenden Ansprüche, wobei die Reaktionskammer (2) mit Reflexionsmitteln, wie etwa einer reflektierenden Oberfläche, wie einer Spiegeloberfläche, zum Erhöhen der Effizienz der Photonenaufzeichnung durch die photoelektrischen Elemente (5) versehen ist.

7. Gasmessvorrichtung (1) nach einem oder mehreren der vorhergehenden Ansprüche, umfassend ein photoelektrisches Referenzelement (5'), das gegen Photonen, insbesondere die aus der Reaktion resultierenden Photonen, geschützt ist, zum Messen eines im Wesentlichen Dunkelstroms, wobei die Anzahl der photoelektrischen Referenzelemente (5') vorzugsweise der Anzahl der photoelektrischen Elemente (5) entspricht.

8. Gasmessvorrichtung (1) nach Anspruch 7, wobei die photoelektrischen Elemente (5) und/oder die photoelektrischen Referenzelemente (5') in einer Linie oder einer Matrix angeordnet sind, vorzugsweise wobei die Anordnung für die photoelektrischen Elemente (5) und/oder die photoelektrischen Referenzelemente (5') gleich ist, vorzugsweise wobei einem photoelektrischen Element (5) der ersten Anordnung ein jeweiliges photoelektrisches Referenzelement (5') der zweiten Anordnung zugeordnet ist und diese immer ein Elementpaar ausbilden, ferner vorzugsweise wobei die photoelektrischen Elemente (5) und die photoelektrischen Referenzelemente (5') während des Betriebs verwendet werden, um eine Differenzmessung durchzuführen, ferner vorzugsweise wobei ein Eingang der Verarbeitungsschaltung (31) mit dem photoelektrischen Element (5) verbunden ist und/oder das photoelektrische Referenzelement (5') mit einer Luftisolierung zum Minimieren parasitärer Widerstände versehen ist.

9. Verfahren zum Durchführen einer Messung in Bezug auf die Konzentration eines Gases, wie etwa Stickoxide, wie etwa Stickstoffdioxide, in einem Gasgemisch wie etwa Luft, unter Anwenden von Messdaten, die mittels einer Gasmessvorrichtung (1), wie in einem oder mehreren der vorhergehenden Ansprüche 1 bis 8 beansprucht, erhalten wurden, wobei das Verfahren die folgenden Schritte umfasst:

   - Zuführen eines Gasgemisches in eine Reaktionskammer (2) ;
   - Inberührungbringen des Gasgemisches mit einer Reaktionsflüssigkeit in der Reaktionskammer (2);
   - Ermöglichen, dass das Gasgemisch mit der Reaktionsflüssigkeit zum Zweck des Erzeugens von Photonen reagiert;
   - Ermöglichen, dass das Gasgemisch die Reaktionskammer (2) mittels des Gasausstoßmittels verlässt;
   - Einfangen der erzeugten Photonen mit einem photoelektrischen Element (5) zum Zweck des Umwandelns von Photonen in elektrische Energie;
   - Umwandeln der elektrischen Energie mit der Verarbeitungsschaltung (31), wie etwa einer Verstärkungsschaltung für eine Abtastschaltung, zum Zweck des Bereitstellens wenigstens eines Signals zum Abtasten, das einem Wert der freigesetzten Photonen entspricht.

10. Verfahren nach Anspruch 9, umfassend die folgenden Schritte:

   - Ergänzen oder Nachfüllen der Reaktionsflüssigkeit in der Reaktionskammer (2) mittels zusätzlicher Reaktionsflüssigkeit über eine Reaktionsflüssigkeitszufuhr (7) und einen Reaktionsflüssigkeitsausstoß (4).

11. Verfahren nach Anspruch 9 oder 10, umfassend die folgenden Schritte:

   - Anwenden einer Reaktionsflüssigkeit zur Verwendung in einer Reaktion mit minimaler Schaumproduktion, wie etwa einer Lumineszenzflüssigkeit, wie etwa einer, die Luminol enthält.

12. Verfahren nach einem oder mehreren der Ansprüche 9 bis 11, umfassend die folgenden Schritte:

   - Bestimmen der Höhe der Gasmessvorrichtung mittels der Umgebungstemperatur und/oder des Umgebungsdrucks.

13. Verfahren zum Verarbeiten einer Messung in Bezug auf eine Konzentration eines Gases, wie etwa Stickoxide, wie etwa Stickstoffdioxide, in einem Gasgemisch wie etwa Luft, unter Anwenden von Messdaten, die mittels einer Gasmessvorrichtung (1), wie in einem oder mehreren der vorhergehenden Ansprüche 1 bis 8 beansprucht, erhalten wurden, wobei das Verfahren die folgenden Schritte umfasst:

   - Empfangen des wenigstens einen abgetasteten Signals, das mittels eines Abtastens des Signals zum Abtasten erhalten wurde;
   - Bestimmen, auf der Basis des abgetasteten Signals, der elektrischen Energie, die von dem wenigstens einen photoelektrischen Element kommt, wobei das Signal zum Abtasten zum Durchführen weiterer Schritte geeignet ist, die zum Bestimmen einer Konzentration eines Gases in einem Gasgemisch auf der Basis der elektrischen Energie angemessen sind.

14. Verfahren nach Anspruch 13, umfassend Schritte zum Kompensieren der Konzentration des Gases in dem Gasgemisch, wobei eines oder mehrere der folgenden kompensierbaren Phänomene kompensiert werden:

   - Dunkelstrom, wobei die Kompensation unter anderem von der Temperatur abhängt;
   - chemische Wirksamkeit eines Reaktionsgemisches in der Gasmessvorrichtung, wobei die Kompensation unter anderem von der Temperatur und dem Luftdruck abhängt;
   - Kompensation der Umwandlung von Teilchendichte zu Partialdruck, wobei die Kompensation unter anderem von der Temperatur und dem Luftdruck abhängt;
   - Kompensation der Umwandlung von Partialdruck zu der Konzentration, wobei die Kompensation unter anderem von der Temperatur und dem Luftdruck abhängt.

**EP 2 946 192 B1**

**Revendications**

1.  Dispositif de mesure de gaz (1) mobile, tel que pouvant être envoyé dans l'espace, pour effectuer une mesure relative à une concentration d'un gaz, tel que des oxydes d'azote, tel que des dioxydes d'azote, dans un mélange gazeux tel que l'air, comprenant :

    - une chambre de réaction (2) adaptée pour contenir un mélange réactionnel liquide afin de mettre le gaz en contact avec le mélange réactionnel liquide ;
    - un moyen d'alimentation en gaz (3), tel que comprenant un tuyau d'alimentation en mélange gazeux, pour alimenter en mélange gazeux la chambre de réaction (2) ;
    - un moyen d'évacuation de gaz (4), tel que comprenant un tuyau d'évacuation de mélange gazeux, pour évacuer du gaz de la chambre de réaction (2) ;
    - au moins un élément photoélectrique (5), tel qu'une photodiode, pour convertir des photons, qui sont libérés lors d'une réaction entre le gaz et le mélange réactionnel liquide, en énergie électrique ;
    - un circuit de traitement (31), tel qu'un circuit d'amplification pour un circuit d'échantillonnage, afin de fournir un signal d'échantillonnage correspondant à une valeur des photons libérés, dans lequel le moyen d'alimentation en gaz est agencé pour introduire le gaz dans la chambre de réaction (2) au niveau ou à proximité de la face inférieure ou au moins de telle sorte qu'un trajet d'ascension du gaz dans la chambre de réaction soit possible sous l'influence de la force d'Archimède.

2.  Dispositif de mesure de gaz (1) selon la revendication 1, dans lequel le dispositif (1) comprend un moyen de régulation de l'humidité, tel que comprenant un moyen d'étanchéité, tel qu'une cascade au moins double de joints, pour rendre étanche des parties du dispositif (1), et/ou dans lequel le dispositif (1) comprend un moyen d'absorption d'humidité.

3.  Dispositif de mesure de gaz (1) selon la revendication 1, dans lequel le dispositif de mesure de gaz (1) comprend un compartiment électronique (24) pour fournir une protection contre la lumière, les fluides et/ou le rayonnement électromagnétique, dans lequel la chambre de réaction, l'élément photoélectrique et/ou le circuit de traitement sont agencés dans le compartiment électronique (24), de préférence dans lequel le compartiment électronique est fourni avec un joint d'étanchéité, de préférence au moyen d'un moyen d'étanchéité qui est adapté pour fournir un joint sur la chambre de réaction et sur une paroi du compartiment électronique (24).

4.  Dispositif de mesure de gaz (1) selon une ou plusieurs des revendications précédentes, comprenant une enceinte électronique pour fournir une enceinte d'au moins une partie d'enregistrement de l'élément photoélectrique (5), laquelle enceinte est de préférence agencée à l'intérieur du compartiment électronique, dans lequel l'enceinte électronique, qui comprend de préférence un manchon rétractable et/ou un produit d'étanchéité, renferme l'électronique afin de protéger l'électronique contre les effets de fluide et/ou de lumière.

5.  Dispositif de mesure de gaz (1) selon une ou plusieurs des revendications précédentes, dans lequel le dispositif (1) comprend au moins deux éléments photoélectriques (5) qui sont connectés en parallèle.

6.  Dispositif de mesure de gaz (1) selon une ou plusieurs des revendications précédentes, dans lequel la chambre de réaction (2) est fournie avec un moyen réfléchissant, tel qu'une surface réfléchissante, tel qu'une surface spéculaire, pour augmenter l'efficacité de l'enregistrement de photons par les éléments photoélectriques (5) .

7.  Dispositif de mesure de gaz (1) selon une ou plusieurs des revendications précédentes, comprenant un élément photoélectrique de référence (5') qui est protégé des photons, en particulier des photons résultant de la réaction, pour mesurer un courant sensiblement d'obscurité, dans lequel le nombre d'éléments photoélectriques de référence (5') est de préférence égal au nombre d'éléments photoélectriques (5).

8.  Dispositif de mesure de gaz (1) selon la revendication 7, dans lequel les éléments photoélectriques (5) et/ou les éléments photoélectriques de référence (5') sont agencés en ligne ou en matrice, de préférence dans lequel l'agencement est le même pour les éléments photoélectriques (5) et/ou des éléments photoélectriques de référence (5'), de préférence dans lesquel un élément photoélectrique (5) du premier agencement se voit attribuer un élément photoélectrique de référence respectif (5') du second agencement, et ceux-ci forment toujours une paire d'éléments, en outre de préférence dans lequel les éléments photoélectriques (5) et les éléments photoélectriques de référence (5') sont utilisés pendant le fonctionnement pour effectuer une mesure différentielle, de préférence en outre dans lequel une entrée du circuit de traitement (31) est connectée à l'élément photoélectrique (5) et/ou l'élément photoélectrique de référence (5') est fourni avec une isolation à l'air pour minimiser les résistances parasites.

16

9. Procédé pour effectuer une mesure relative à la concentration d'un gaz, tel que des oxydes d'azote, tel que des dioxydes d'azote, dans un mélange gazeux tel que l'air, en appliquant des données de mesure obtenues au moyen d'un dispositif de mesure de gaz (1) selon l'une ou plusieurs des revendications précédentes 1 à 8, le procédé comprenant les étapes consistant à :

- alimenter en mélange gazeux une chambre de réaction (2) ;
- amener le mélange gazeux en contact avec un liquide réactionnel dans la chambre de réaction (2) ;
- laisser le mélange gazeux réagir avec le liquide réactionnel afin de générer des photons ;
- laisser le mélange gazeux sortir de la chambre de réaction (2) au moyen du moyen d'évacuation de gaz ;
- capturer les photons générés avec un élément photoélectrique (5) afin de convertir des photons en énergie électrique ;
- convertir l'énergie électrique avec le circuit de traitement (31), tel qu'un circuit d'amplification pour un circuit d'échantillonnage, afin de fournir au moins un signal d'échantillonnage correspondant à une valeur des photons libérés.

10. Procédé selon la revendication 9, comprenant les étapes consistant à :

- compléter ou réapprovisionner le liquide réactionnel dans la chambre de réaction (2) au moyen d'un liquide réactionnel supplémentaire par l'intermédiaire d'une alimentation en liquide réactionnel (7) et d'une évacuation de liquide réactionnel (4).

11. Procédé selon la revendication 9 ou 10, comprenant les étapes consistant à :

- appliquer un liquide réactionnel à utiliser dans une réaction avec une production de mousse minimale, tel qu'un liquide luminescent, tel que contenant du luminol.

12. Procédé selon une ou plusieurs des revendications 9 à 11, comprenant l'étape consistant à :

- déterminer l'altitude du dispositif de mesure de gaz au moyen de la température et/ou de la pression ambiantes.

13. Procédé de traitement d'une mesure relative à une concentration d'un gaz, tel que des oxydes d'azote, tel que des dioxydes d'azote, dans un mélange gazeux tel que l'air, en appliquant des données de mesure obtenues au moyen d'un dispositif de mesure de gaz (1) selon l'une ou plusieurs des revendications précédentes 1 à 8, le procédé comprenant les étapes consistant à :

- recevoir l'au moins un signal échantillonné obtenu au moyen d'un échantillonnage du signal d'échantillonnage ;
- déterminer sur la base du signal échantillonné l'énergie électrique provenant de l'au moins un élément photoélectrique, lequel signal d'échantillonnage est adapté pour effectuer d'autres étapes appropriées pour déterminer une concentration d'un gaz dans un mélange gazeux sur la base de l'énergie électrique.

14. Procédé selon la revendication 13, comprenant des étapes pour compenser la concentration du gaz dans le mélange gazeux, un ou plusieurs des phénomènes compensables suivants étant compensés :

- le courant d'obscurité, la compensation dépendant, entre autres facteurs, de la température ;
- l'efficacité chimique d'un mélange réactionnel dans le dispositif de mesure de gaz, la compensation dépendant, entre autres facteurs, de la température et de la pression d'air ;
- la compensation de la conversion de la densité de particules en pression partielle, la compensation dépendant, entre autres facteurs, de la température et de la pression d'air ;
- la compensation de la conversion de la pression partielle en concentration, la compensation dépendant, entre autres facteurs, de la température et de la pression d'air.

Fig. 1

Fig. 2

## Fig. 3

## Fig. 5

Fig. 4

Fig. 6

Fig. 7

EP 2 946 192 B1

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004113892 A1